# EUROPEAN PATENT SPECIFICATION

(11) **EP 0 383 417 B1**
(45) Date of publication and mention of the grant of the patent: **27.12.1995**
(21) Application number: 90250043.8
(22) Date of filing: 13.02.1990
(51) Int. Cl.: C12N 9/64, C12N 15/58, C12N 1/21, A61K 38/48

(54) **Vampire bat salivary Plasminogen activator vPA-alpha 1**
Fledermausspeichel-Plasminogenaktivator vPA-alpha 1
Activateur du plasminogène salivaires de chauve-souris vPA-alpha 1

(30) Priority: 13.02.1989 DE 3904580; 30.05.1989 DE 3917949
(43) Date of publication of application: 22.08.1990
(73) Proprietor: SCHERING AKTIENGESELLSCHAFT, 13353 Berlin (DE)
(72) Inventor: Baldus, Berthold, D-1000 Berlin 31 (DE); Donner, Peter, D-1000 Berlin 41 (DE); Schleuning, Wolf-Dieter, D-1000 Berlin 20 (DE); Alagon, Alejandro, Mor. 62271 (MX); Boidol, Werner, D-1000 Berlin 31 (DE); Krätzschmar, Jörn Reiner, D-1000 Berlin 30 (DE); Haendler, Bernhard Jacques, D-1000 Berlin 19 (DE); Langer, Gernot, D-1000 Berlin 21 (DE)

(56) References cited:
- EP-A- 0 352 119
- JOURNAL OF BIOLOGICAL CHEMISTRY, vol. 264, no. 30, October 1989, pages 17947-17952; S.J. GARDELL et al.: "Isolation, characterization and cDNA cloning of avampire bat salivary plasminogen activator"
- BLOOD, vol. 43, no. 3, March 1974, pages 317-326; T. CARTWRIGHT: "Theplasminogen activator of vampire bat saliva"

## Description

The present invention relates to a novel thrombolytic, processes for its isolation, and its pharmaceutical usage.

Thromboses are produced by the formation of a blood clot in blood vessels. One distinguishes between venous thromboses including pulmonary embolisms and arterial thromboses including acute myocardial infarction.

Pulmonary embolism and cardiac infarction are life-threatening events requiring immediate medical intervention.

Besides various invasive methods, a popular form of therapy for arterial and venous thromboses has evolved in recent years in the form of enzymatic thrombolysis with plasminogen activators. These substances, called thrombolytics, convert plasminogen, the inactive proenzyme of the fibrinolysis system in the blood, into the active proteolytic enzyme, plasmin. Plasmin, in turn, dissolves the fibrous substance, fibrin, which is a substantial component of a blood clot; this leads to reopening of the blocked vessels and restoration of blood flow. However, plasmin is a relatively unspecific protease, i.e. once formed in the blood, it destroys, by proteolysis, components in the blood indispensable for intact hemostasis (e.g. fibrinogen) and thereby induces under certain circumstances dangerous risks of hemorrhaging.

The thrombolytics of the first generation, streptokinase and urokinase, are compounds which, once injected into the circulation, systemically convert plasminogen into plasmin and induce systemic proteolysis. Therefore thrombolysis therapies with these substances are frequently accompanied by complications due to hemorrhage. The fibrin-specific thrombolysis, developed thereafter, wherein recombined plasminogen activators of the tissue type, called t-PA for the sake of brevity, are employed, was supposed to lead out of this dilemma. In the blood circulation, t-PA has an only low affinity to plasminogen. However, in the presence of the fibrous substance, fibrin, with which it reacts by way of specific binding sites, this affinity is increased by a multiple, resulting in plasmin formation on the surface of the thrombus. This concept could be verified in vitro and in animal experiments; the clinical studies, however, show that large amounts of t-PA are needed in order to bring about rapid dissolution of a coronary thrombosis.

However, if doses of t-PA of such magnitude are infused, this will lead to a systemic proteolysis accompanied by a relative risk of hemorrhaging, in a similar way as in the cases of streptokinase and urokinase. Therefore, one speaks nowadays of a relative fibrin specificity of t-PA. The cause therefore lies in an essential characteristic of t-PA: This molecule is an active protease which, under favorable conditions (high enzyme concentration, long exposure time, high substrate concentration, optimal pH and ion environment), will convert plasminogen into plasmin even in the absence of fibrin. All of these conditions are met in a present clinical standard therapy with t-PA.

The publication of T. Cartwright describes plasminogen activators, called desmokinase, from saliva of the vampire. The material causes rapid lysis of both plasminogen - rich blood clots and fibrin plates. The material is inactive when applied to fibrin plates without active plasminogen.

The material is able to cause rapid lysis of whole blood clots. The author assumes that no other proteolytic proteins are present. The two stage casein test is useless for characterisation. There are differences between the way in which the material reacts and the mechanism by which urokinase is able to activate plasminogen.

The man skilled in the art cannot find any technical teaching for isolation, purification and characterisation of a thrombolytic agent in the publication T. Cartwright (1974) Blood **43**:317.

The technique used by T. Cartwright comprises a gel filtration supplying impure material. T. Cartwright characterises the found agents by a negative list showing what functions the material do not have and features they are missing.

During a search for more specific plasminogen activators fulfilling the criterion of fibrin specificity, a new, natural compound exhibiting fibrinolytic activity has been found, called v-PA.

The EP 0 352 119 describes vampire bat salivary plasminogen activators (thrombolytic active agents, v-PA) which are isolated from the saliva of the salivary glands of Desmodus rotundus. The agents are native- or recombinant-derived purified proteins which catalyse formation of plasmin which is an enzyme hydrolysing peptides. Plasmin converts fibrin to soluble products. The v-PAs are distinct from t-PA and urokinase by several structural and functional criteria. Unlike t-PA, the v-PAs do not contain the kringle 2 domain and plasmin-sensitive processing site. Equimolar quantities of these v-PAs and t-PA are similarly effective when monitored for their ability of preformed plasma clots. The activity towards plasminogen is stimulated in the presence of fibrin cofactor.

Three distinct species corresponding to first full-length, second finger-minus and third finger / EGF-minus forms are described. They are referred to as v-PAα2; v-PAβ and v-PAγ. The full-length species, namely v-PAα2 has a sequence of 441 amino acids. The mature protein starts with the N-terminus Ala · Tyr · Gly · Val · ... The preprotein has a signal sequence of 36 amino acids identical for all three forms.

The problem of the invention is to supply a further effective plasminogen activator isolatable from the vampire bat salivary.

Surprisingly a further plasminogen activator has been found.

The problem is solved by a substantially pure protein which is thrombolytic, activates plasminogen in the presence of fibrin but substantially not in the presence of fibrinogen, and is allosteric, said protein is a v-PAα1 having the sequence of Figure 16.

A further embodiment of the invention is an isolated DNA encoding a protein according to the invention having the nucleotide sequence of Figure 16.

Preferred is a micro-organism transformed with a vector comprising DNA according to the invention.

Further the invention comprises a method of producing v-PAα1 comprising expressing DNA encoding v-PAα1 in the micro-organism according to the invention.

Preferred is a substantially pure protein according to the invention which has the N-terminal amino acid sequence Ala · Tyr · Gly · Val · Ala · ...

More preferred is a substantially pure protein according to the invention which has the N-terminal amino acid sequence Ala · Tyr · Gly · Val · Ala · Cys · Lys · Asp · Glu · Ile · Thr · Gln · Met · Thr · Tyr · Arg · ...
· The invention concerns the thrombolytically active agent v-PA from the saliva of bats of the genus Desmodus spec., distinguished by the following characteristic properties:
· A cDNA was discovered from a salivary gland cDNA bank which codes for different v-PA protein:
   v-PA α₁: High-molecular form consisting of a finger domain, an EGF (epidermal growth factor) domain, a Kringel domain, and a protease domain (Example 11).
· The main protein band of the active agent v-PA α₁ shows a molecular weight of 43,000 ± 2,000 (Example 1), in a sodium dodecyl sulfate gel electrophoresis under nonreducing conditions.
· The active agent reacts with ³H-diisopropyl fluorophosphate and thus is a serine protease (Example 7).,
· The active agent v-PA α₁ hydrolyzes the chromogenic peptides: S-2288 (H-D-Ile-Pro-Arg-pNA) and S-2444 (<Glu-Gly-Arg-pNA), but not the chromogenic peptide S-2251 (H-D-Val-Leu-Lys-pNA) (Example 9).
· The active agent v-PA α₁ produces lysis halos on fibrin plates, but no on casein plates.
· The active agent v-PA α₁ binds, at a pH of 7.5, to Zn⁺⁺ chelate "Sepharose" (Example 1).
· The active agent lyses, in dependence on the concentration, human whole blood thrombi in vitro (Example 6).
· The active agent v-PA α₁ binds to heparin-"Sepharose" and can again be eluted therefrom by suitable solutions (Example 3).
· The active agent binds to immobilized inhibitor which is isolated from the seeds of various Erythrina species and can again be separated therefrom by suitable solutions (Example 2).
· The active agent v-PA α₁ binds to fibrin-"Celite" and can be eluted again by suitable methods (Example 4).
· The active agent binds to hydroxyapatite under certain conditions and can be eluted again with a phosphate-containing buffer (Example 1).
· The active agents v-PA α₁ and v-PA β differ from each other with regard to their N-terminal amino acid sequence (Example 5).
· The active agents v-PA α₁ and v-PA β differ with respect to their amino acid and nucleotide sequence (Example 11).
· The coding sequence of the active agents can be incorporated into a corresponding vector "septan". These express the active agent in suitable eukaryotic cell systems (Example 12).

The thrombolytic active agent v-PA α₁ represents a novel, naturally occurring plasminogen activator which dissolves blood clots in the human body and thus is suitable for treatment of, for example, cardiac infarction. Therefore, the invention comprises a medical agents based on the compounds according to the invention with customary auxiliary agents and excipients.

This agent is found in the saliva of all species of bats of the genus Desmodus spec. in low concentrations and is probably expressed by the cells of the salivary glands of this type of animal. The bats of the genus Desmodus spec. encompass all kinds of bats of the American continent. The genus Desmodus of Central America and Mexico has been the subject of especially thorough investigation.

The invention also relates to several methods for the isolation of the thrombolytic v-PA from the saliva of Desmodus spec. The centrifuged saliva is taken up in a buffer, chromatographed over a Zn⁺⁺ chelate "Sepharose" column, the active agent v-PA is quantitatively eluted after washing of the column, the collected fractions are filtered in the absense of salts over "Superose" 12, and finally are again subjected to gel filtration in the presence of a physiological saline solution.

The invention furthermore concerns medicinal agents based on the compound v-PA, as well as conventional auxiliary agents and excipients.

The superiority of the novel thrombolytic v-PA as compared with t-PA can be derived from Example 6 set forth below.

By substantially pure is meant that a protein is purified, e.g. to a degree that its activity is enhanced significantly over that of the protein in nature. Preferably, the protein will be purified to homogeneity and most preferably will be at least 90 %, especially greater than 95 % pure or higher.

The DNA sequences of this invention can be routinely inserted into the common, e.g. commercially available, vectors such as plasmids, phages etc. useful for expression in, e.g., the common, e.g., commercially available cell lines.

Oligonucleotide probes specific for v-PA sequences will be constructed routinely by selecting sequences in the leader region or the complete sequence which are different from t-PA (see comparison in fig. 17).

Antibodies can also be routinely prepared by normal methods, e.g., by raising them in animals such as goats.

The v-PA of this invention can be administered to mammals, including humans, analogously to the use of the t-PA but with fewer side effects due to the selectivity of v-PA as discussed above. In general, lower doses than otherwise needed for t-PA may be employable. Suitable activities can be routinely determined by employing conventional protocols. Typical indications include those for which thrombolytics are useful, e.g., cardiac infarct, stroke, arterial thrombosis, etc. The usual galenic additives can be employed to produce pharmaceutical formulations.

### Example 1

### Purification and Separation of v-PA α₁ from the Saliva of the Vampire Bat Desmodus rotundus

(a) Zn++ Chelate "Sepharose" Chromatography
   20 ml of saliva is centrifuged at 4 °C and 6,000 g. The supernatant is adjusted to 20 mM Tris pH 7.5/ 100 mM NaCl and 0.05 % "Pluronic" F 68. A chromatographing tube is filled with 8 ml of Zn⁺⁺ chelate "Sepharose" equilibrated with 10 mM Tris pH 7.5 / 100 mM NaCl / 1 mM imidazole / 0.05 % "Pluronic" F 68, and the saliva supernatant is chromatographed thereover. The column is washed with equilibrating buffer. Thereafter, with 10 mM Tris pH 7.5 / 1 M NaCl / 1 mM imidazole / 0.05% "Pluronic" F 68, primarily v-PA α₁ is eluted, which still contains a small proportion of v-PA β (Fig. 1). Then, v-PA β, still containing a small proportion of v-PA α₁, is eluted with a gradient of 1 - 50 mM imidazole in 10 mM Tris pH 7.5 / 100 mM NaCl / 0.05 % "Pluronic" F 68. 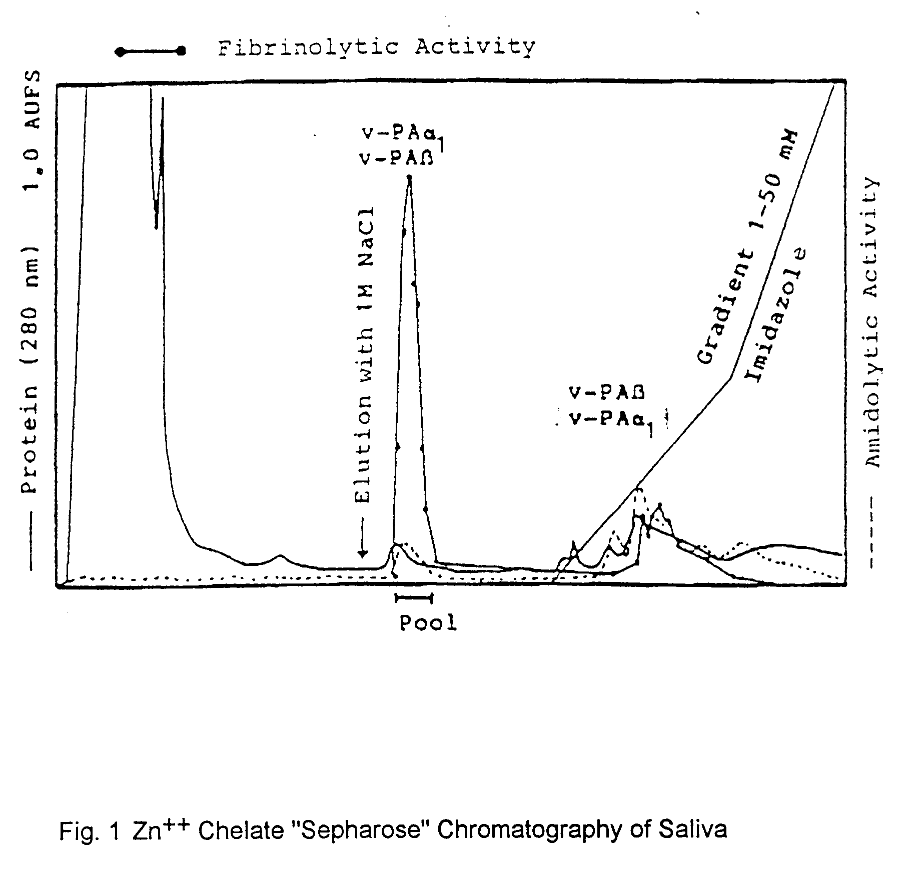
(b) Hydroxyapatite Chromatography
   A chromatographing column is charged with 8 ml of hydroxyapatite and equilibrated with 50 mM Tris pH 8.0 / 500 mM NaCl / 0.01 % "Pluronic" F 68. The pool from Zn⁺⁺ chelate chromatography (Fig. 1) is passed over the hydroxyapatite column and washed with the equilibrating buffer, and the v-PA is eluted with a gradient of 0.5 - 100 mM Na phosphate pH 7.5 in 500 mM NaCl / 0.01 % "Pluronic" F 68 (Fig. 2). The active fractions are pooled. 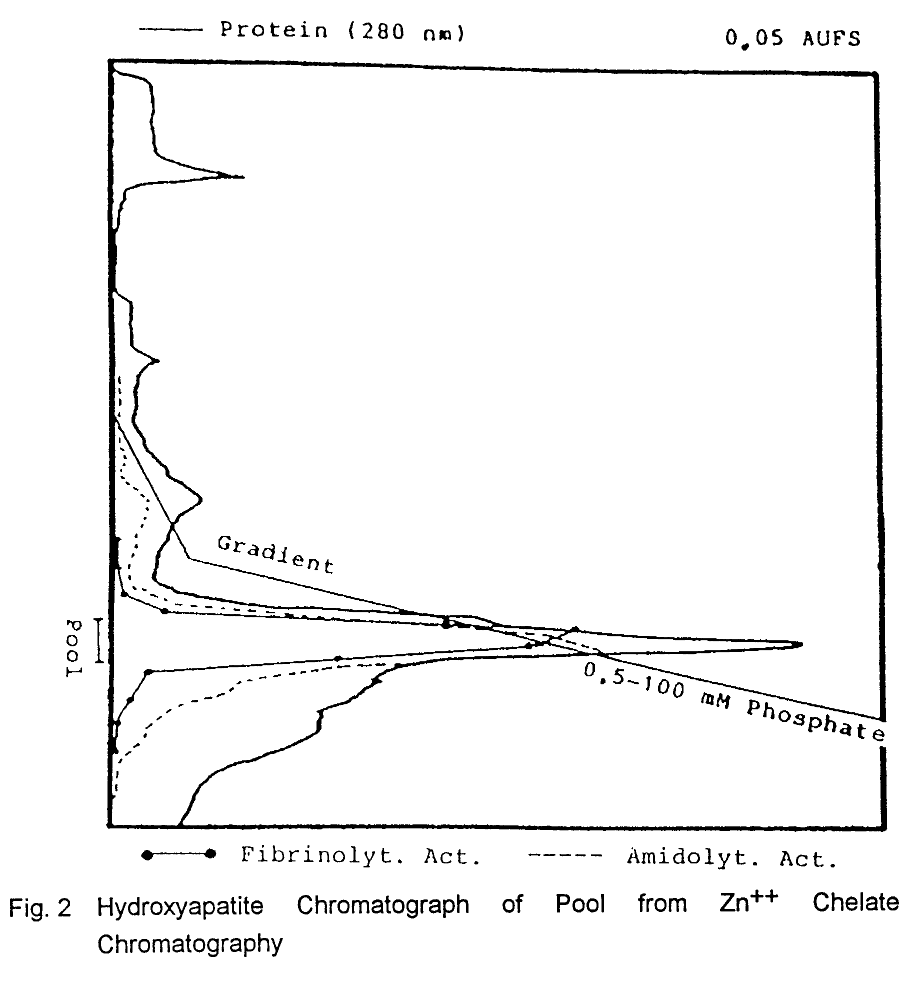
(c) Gel Filtration
   The pool from the hydroxyapatite chromatography (Fig. 2) is dialyzed against 20 mM Tris pH 8.0 / 160 mM NaCl and concentrated to 0.6 ml by freeze-drying. This concentrate is filered with the aid of an FPLC system (Pharmacia) over a "Superose" 12 HR 16/50 column (Fig. 3).

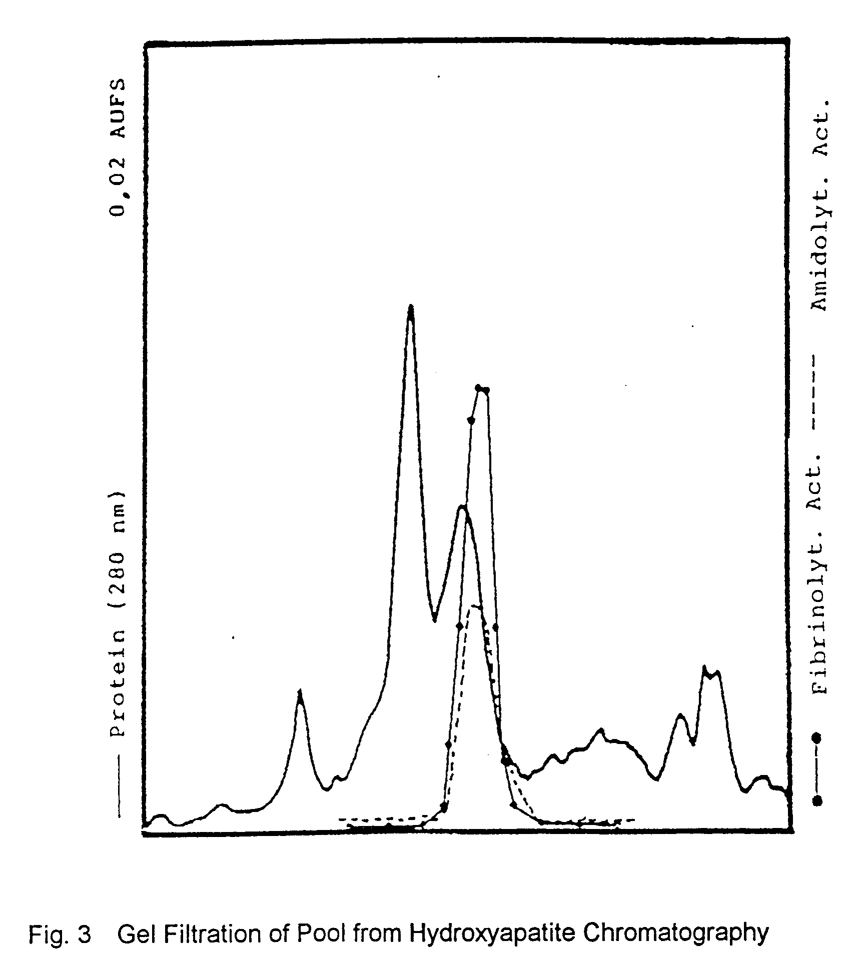

When analyzing a concentrate of the active fractions of the gel filtration with the aid of SDS polyacrylamide gel electrophoresis under nonreducing conditions, and thereafter staining the gel with "Coomassie" blue, a protein band becomes visible having a molecular weight of 43,000 (Fig. 4). 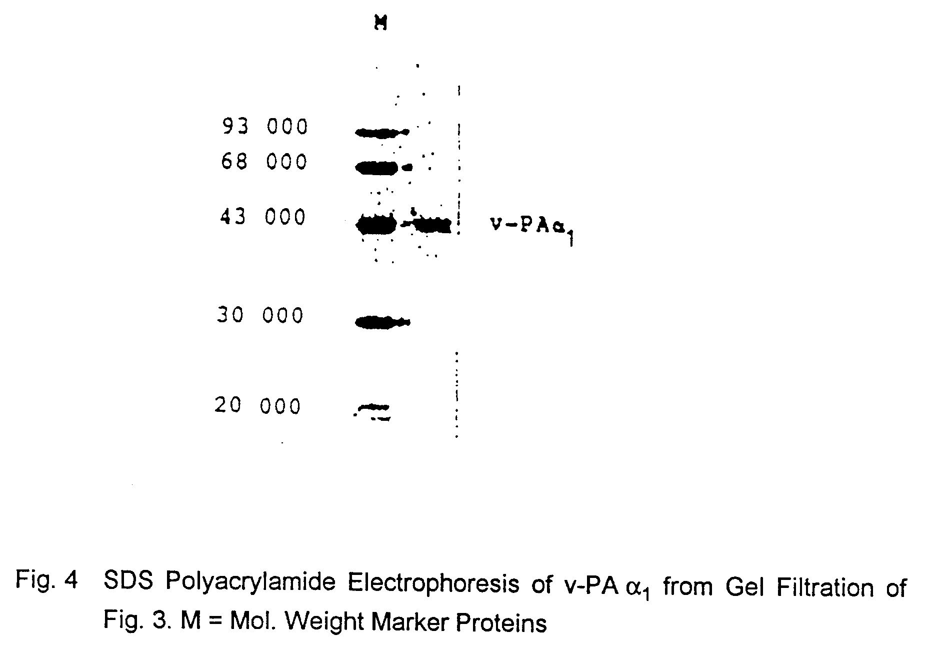
As can be seen from Fig. 4, v-PA α₁ is free of other proteins. Also this purified v-PA α₁ yields a lysis halo with an identical molecular weight of 43,000 in an indicator gel (fibrin zymography). Also, this lysis halo can be found exclusively on a plasminogen-containing fibrin indicator gel, and not on a plasminogen-containing casein indicator gel.

### Example 2

### Binding and Elution of v-PA α₁ to the Trypsin Inhibitor DE-3 from the Seeds of Erythrina latissmia (ETI)

50 mg of ETI (Erytech Services Ltd., Arcadia, South Africa) is coupled in covalent fashion to 6.6 g of activated CH "Sepharose" 4B (Pharmacia) in accordance with the manufacturer's data. With 1 M Tris pH 6.8, 16 ml of saliva is adjusted to pH 7.5 and centrifuged for 10 minutes at 4 °C and 6,000 g.

A chromatographing tube HR 10/2 (Pharmacia) is filled with 2 ml of ETI CH "Sepharose" 4B (see above). With the aid of an FPLC system (Pharmacia), this affinity chromatography is conducted. For this purpose, the saliva supernatant is passed over the ETI CH "Sepharose" 4B and thoroughly washed with 20 mM Tris pH 7.5 / 400 mM NaCl / 0.01 % "Pluronic". Elution is performed with a solution of 20 mM Tris pH 7.5 / 400 mM NaCl / 1.6 M KSCN / 0.01 % "Pluronic" F 68. During this step, the fibrinolytic as well as the amidolytic activity is eluted together with one protein peak. 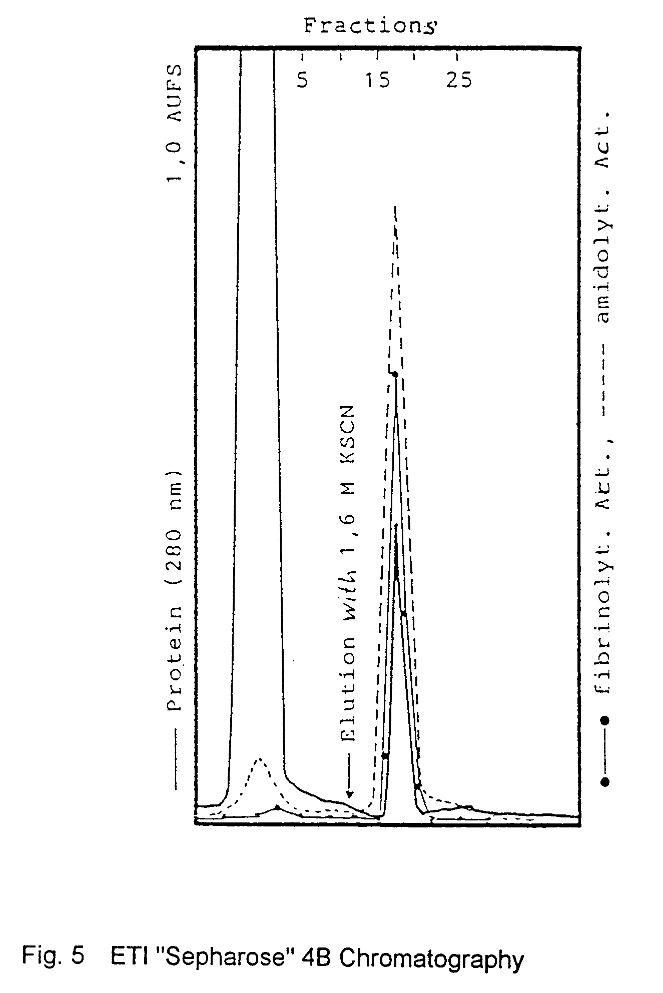
When analyzing this chromatography with the aid of SDS polyacrylamide gel electrophoresis under nonreducing conditions, a strong enrichment of the two v-PA forms v-PA α₁ and v-PA β can be confirmed.

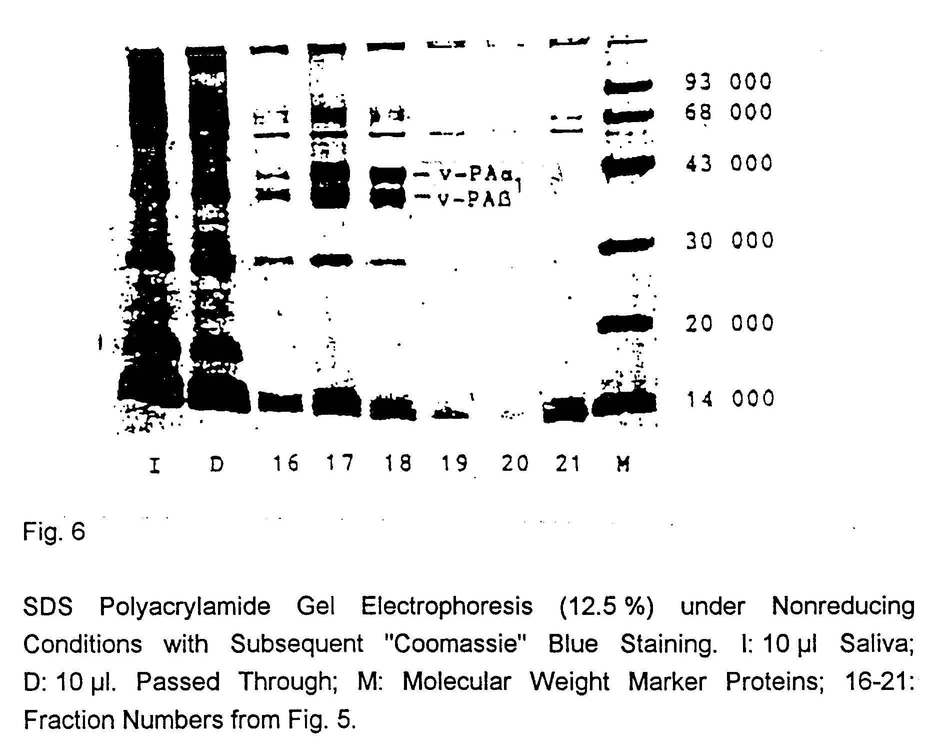

The two molecular v-PA forms occurring in this batch of saliva bind, under the above-mentioned conditions, to ETI-CH "Sepharose" 4B and can be eluted in the presence of 1.6 M KSCN or other chaotropic compounds. This holds true for all forms of the active agent.

### Example 3

### Binding and Elution of Active Agent v-PA α₁ to Heparin "Sepharose"

An unpacked FPLG column (HR 5/5) is filled with one milliliter of heparin "Sepharose" (Pharmacia). Fraction 18 from ETI-CH "Sepharose" 4B chromatography (Example 2, Figs. 5 and 6) is dialyzed against 20 mM Tris pH 7.5 / 50 mM NaCl / 0.01 % "Pluronic" F 68 and chromatographed over heparin "Sepharose" with the aid of an FPLC system (Pharmacia). After application of the dialyzate, the column is washed with 20 mM Tris pH 7.2 / 50 mM NaCl / 0.01 % "Pluronic" F 68, and then eluted with a gradient of 50 - 1,000 mM NaCl in 20 mM Tris pH 7.2 / 0.01 % "Pluronic" F 68 (Fig. 7).

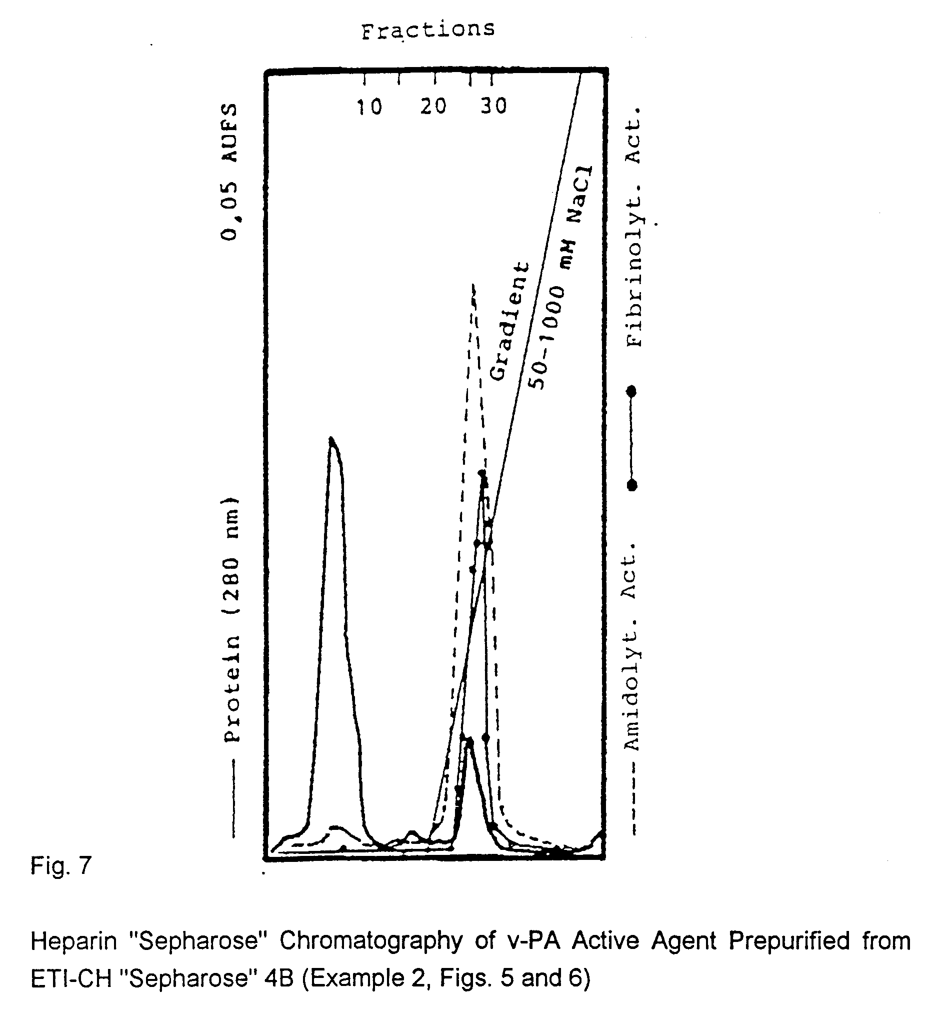

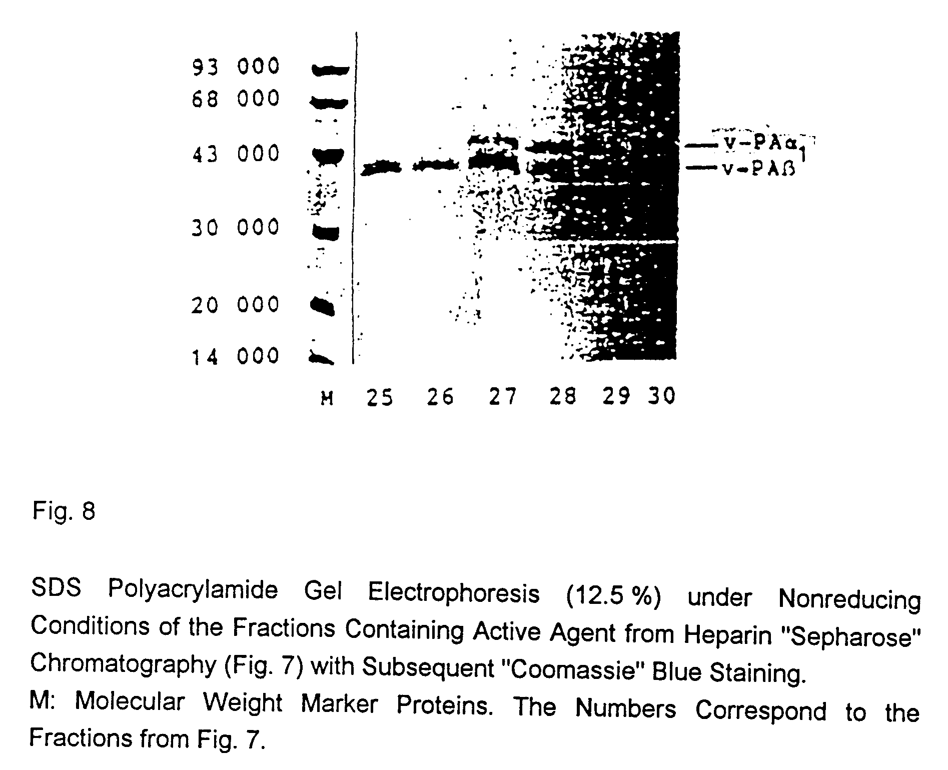

With the aid of heparin "Sepharose" chromatography, it is possible, for example, to bind and elute the molecular forms v-PA α₁ and v-PA β, i.e. to purify them. As can be seen from Figures 7 and 8, the molecular v-PA form, v-PA β, shows a weaker binding to heparin "Sepharose" than the molecular v-PA α₁ form; i.e. for the elution of v-PA β, a lower NaCl concentration is adequate than is needed for elution of v-PA α₁.

Thus, with the aid of the chromatographical methods disclosed in this example, it is possible to separate the two v-PA forms from each other.

### Example 4

### Binding and Elution of Active Agent v-PA α₁ to Fibrin "Celite"

The affinity medium fibrin "Celite" is prepared according to known directions (Husain, S., Lipinski, B and Gurewich, V. in Proc. Natl. Acad. Sci. USA 78 : 4265-4269, 1981). One milliliter of the fibrin "Celite" material is filled into a chromatographing column HR 5/5 (Pharmacia).

Fraction 17 from ETI-CH "Sepharose" 4B chromatography (Example 2, Figs. 5 and 6) is dialyzed against 20 mM Tris pH 8.0 / 20 mM NaCl / 0.01 % "Pluronic" F 68, and chromatographed over fibrin "Celite" with the aid of an FPLC system (Pharmacia). After application of the dialyzed fraction, washing is performed with 20 mM Tris pH 8.0 / 20 mM NaCl / 0.01 % "Pluronic" F 68, and the thus-found active agent is eluted with a gradient of 20 - 1,000 mM NaCl in 20 mM Tris pH 8.0 / 0.01 % "Pluronic" F 68 (Fig. 9).

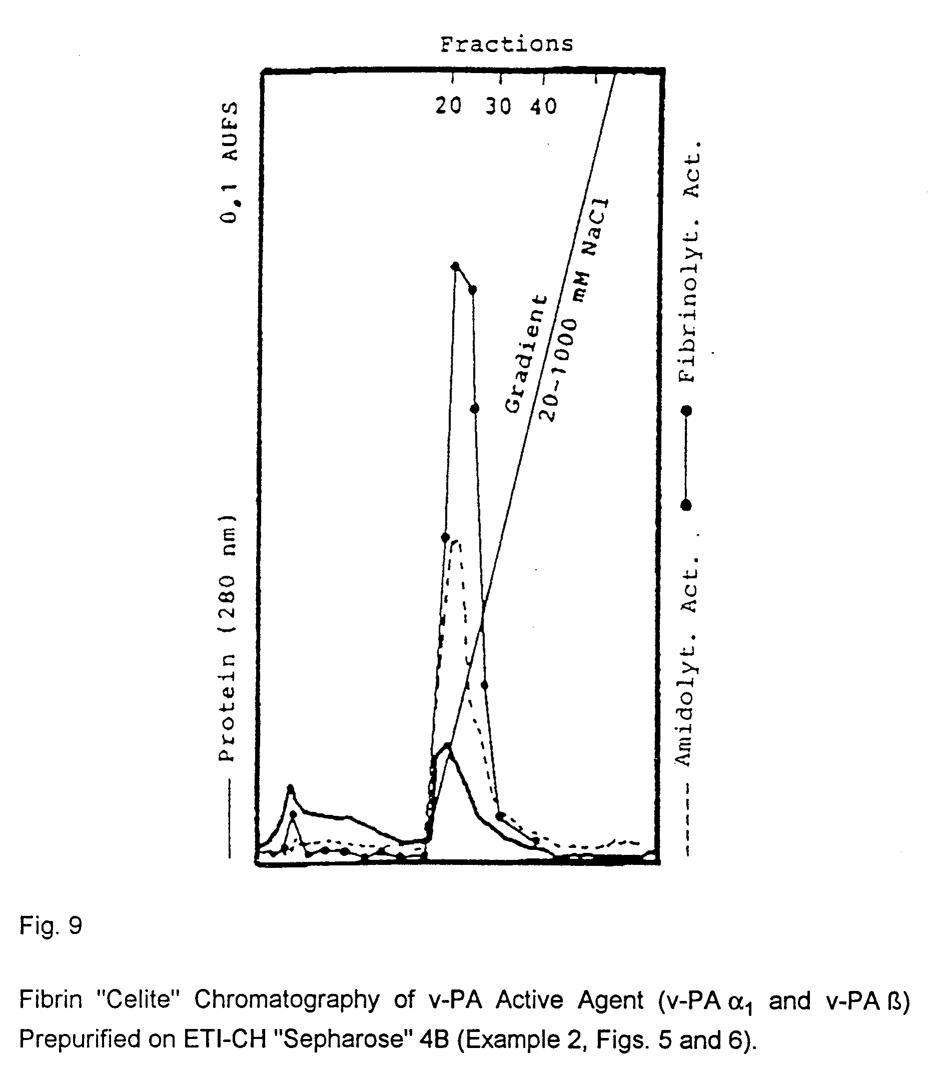

The v-PA-containing fractions are subjected to SDS polyacrlylamide gel electrophoresis (Fig. 10).

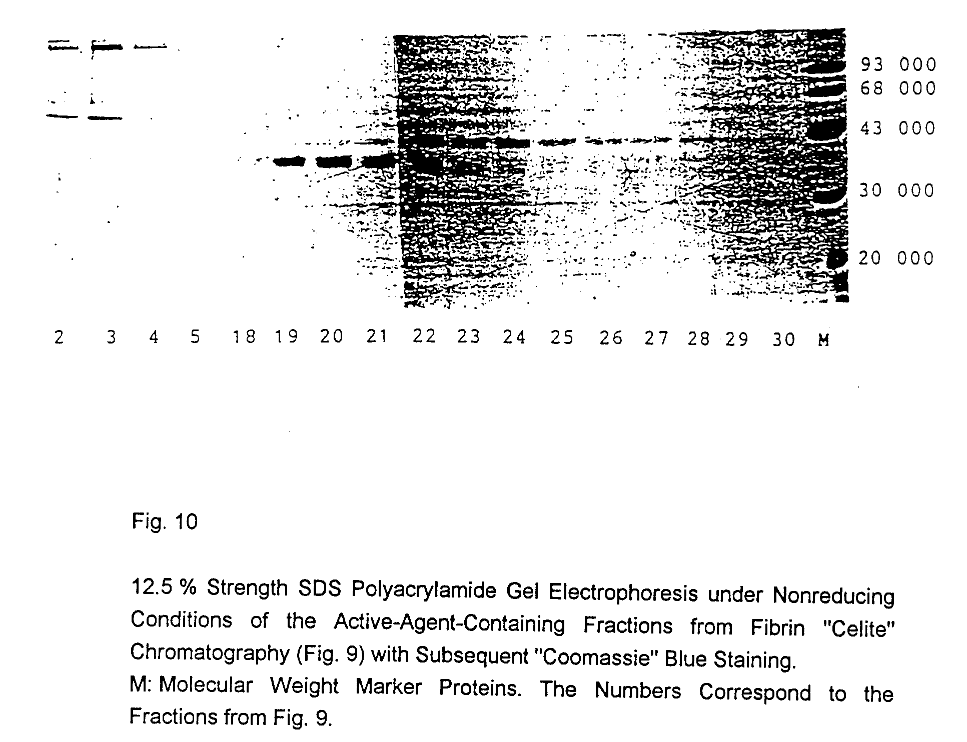

The active agents v-PA α₁ and v-PA β bind, at low NaCl concentrations, to fibrin "Celites" and can be again eluted by increasing the NaCl concentration in the buffer. During this step, one can observe, that v-PA β obviously shows weaker binding to fibrin "Celites" than v-PA α₁ since, in contrast to v-PA β, the v-PA α₁ form elutes only at higher NaCl concentrations. Thus, this purifying method can also be utilized for separation of v-PA β and v-PA α₁.

### Example 5

### N-Terminal Amino Acid Sequence Analysis of the Purified v-PA Forms v-PA α₁ and v-PA β

Fractions containing purified v-PA α₁ (Example 1, Fig. 4) or purified v-PA β are subjected, after dialysis against a suitable buffer, to a 12.5 % polyacrylamide gel electrophoresis under nonreducing conditions. After electrophoresis has taken place, the gel is removed from the electrophoresis has taken place, the gel is removed from the electrophoretic apparatus and transferred according to a conventional method (Matsudaira, P. in J. Biol. Chem. 262 : 10035-10038, 1987) to a polyvinylidene difluoride membrane (Immobilon, Millipore Corp. USA). The corresponding protein band is excised with a scalpel and, in an automatic 477 A Protein Sequencer / 120 A Analyzer (Applied Biosystems USA), the N-terminal amino acid sequence is directly determined. The analyses are repeatedly performed. The sequences set out below are found:
v-PAα1
Ala Tyr Gly Val Ala X Lys X Glu Ile Thr Gln Met Thr Tyr Arg
V-PAβ
Ala Tyr Gly Gly X Ser Glu Leu Arg Cys Phe Asn Gly Gly Thr X Gln Ala Ala
(X = not clearly determined)
With the aid of these thus-determined sequences, a cDNA bank was screened from the saliva gland of Desmodos rotundus (Example 11).

### Example 6

### Comparison of Thrombolytic Activity of v-PA α₁ with that of t-PA

The thrombolytic activity of the novel active agent is examined in comparison with t-PA in a newly developed "Micro-Clot-Lysis Assay" (MCLA). Respectively 100 »l of fresh human whole blood is pipetted into the holes of microtiter plates, combined with respectively 10 »l of a clotting agent ("Thromborel", Behring-Werke), and incubated at 37 °C for 1 hour. The resultant blood clots are washed twice with phosphate-buffered sodium chloride solution (BPS) before they are combined with 100 »l of autologous plasma previously obtained by centrifuging. The plasma is subsequently combined with t-PA and, respectively, v-PA in concentrations of 1.56 - 12.5 U/ml. The thus-treated microtiter plates are stored for 18 hours at 37 °C in a moist chamber. After elapse of the incubating period, the plates are shaken on a vibrating table (Red Rotor), and thereafter a plasma specimen is withdrawn from each hole, diluted with twice-distilled water 1 : 6 (leads to rupturing of the red blood cells liberated by lysis), and the thus-released red blood pigment is determined photometrically at 492 nm in a microtiter plate photometer. 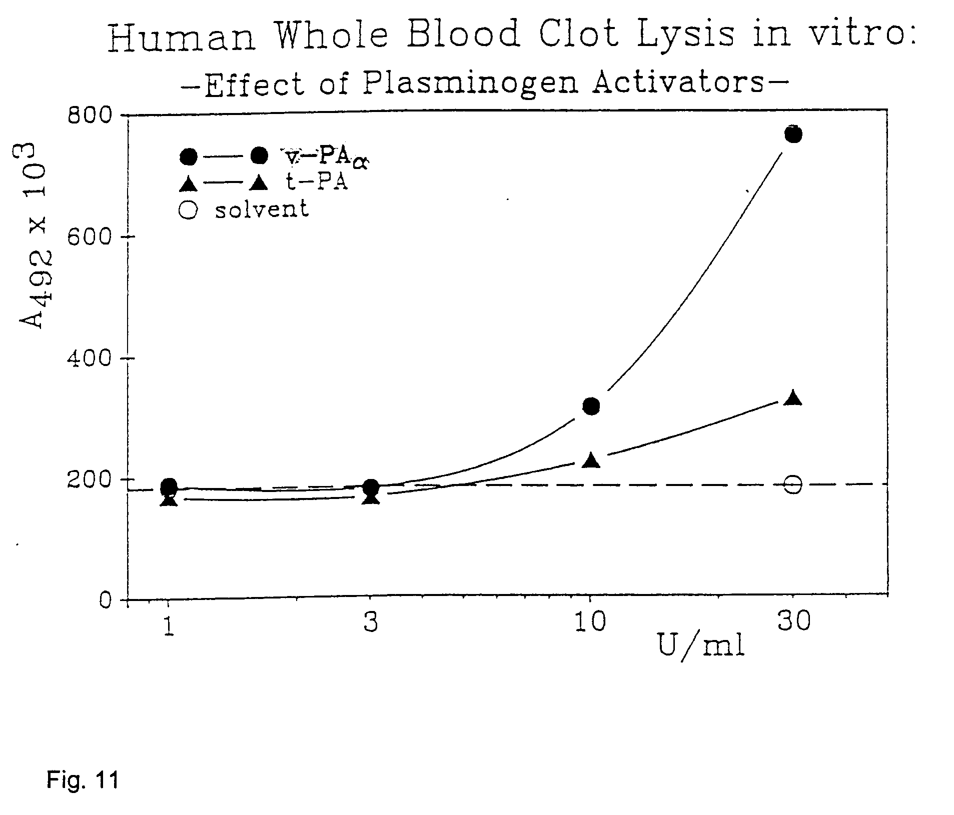
The result of the test can be seen from Figure 11: In comparison with a standard t-PA, equieffective concentrations of the novel active agent lyse (as determined in the International Clot-Lysis Assay) human whole blood thrombi better than t-PA. Even about 3 U/ml of the novel active agent v-PA β shows, in the described test model, a significant thrombolytic effect whereas 3U t-PA shows no results deviating from the control.

In a similar experiment, the time dependency of the human whole blood thrombolysis by t-PA and v-PA is investigated.

Respectively 6.25 U and 12.5 U of t-PA and of v-PA are incubated in the above-described system for 23 hours at 37 °C. After 15 hours, a sample is withdrawn every 2 hours and prepared as described for the photometric test. The result is illustrated in Figure 12. 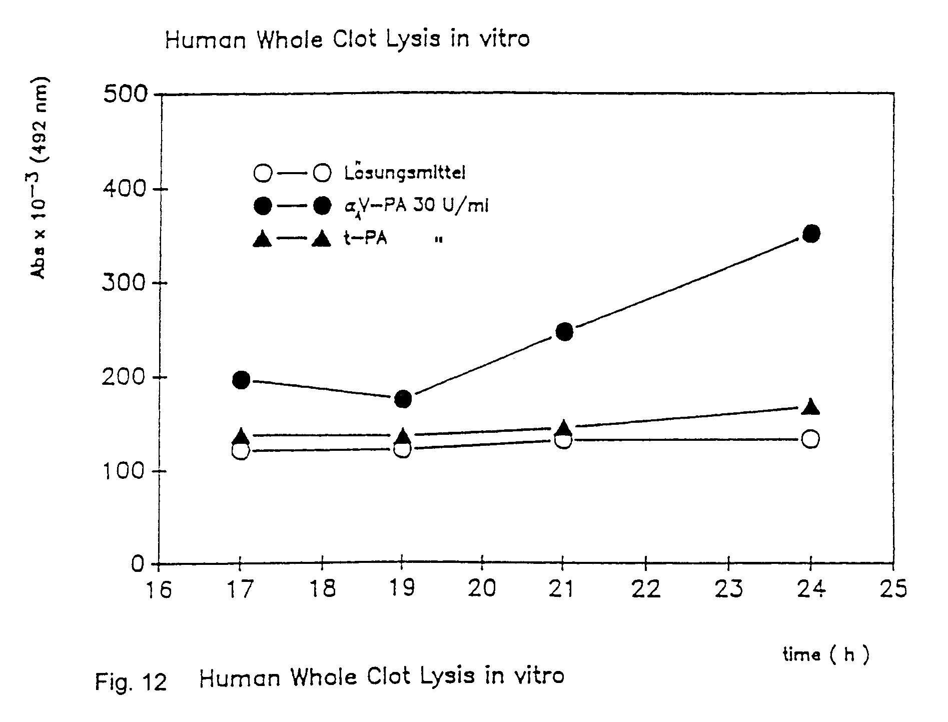
The beginning of thrombolysis, measured as release of red blood cells from the thrombus, is clearly more rapid with the active substance than with t-PA. 6,25 Units of the new active substance reveale an enhanced thrombolysis after 23 hours as the double activity of t-PA (12,5 Units).

### Example 7

### Serine Proteases React Specifically with the "Active Site Titrant" Diisopropyl Fluorophosphate (DIFP)

For this purpose, the active agent is buffered to pH8.0 and mixed in a ratio of 1 : 20 with 3H-DIFP in propylene glycol. The batch is incubated overnight at room temperature. An aliquot of the batch is separated on a 12.5 % SDS gel under nonreducing conditions. The gel is then incubated for 30 minutes at room temperature in "Amplify" (Amersham) and subsequently dried. Then the gel is exposed for 4 days and subsequently the X-ray film is developed. Serine proteases which have the radioactively labeled DIFP incorporated therein become visible on the film as blackened bands (Fig. 13). t-PA yields, for example, a slightly blackened main band with the apparent molecular weight of 68,000 ± 5,000; in contrast thereto, the active agent shows a strongly blackened main band of 39,000 ± 2,000 molecular weight (v-PA β) and a blackened band at 43,000 ± 2000 (v-PA α₁).

A control gel obtained under identical electrophoretic conditions is washed with 1 % "Triton" X-100 and subsequently placed on a fibrin- and plasminogen-containing indicator gel. The position of the activities of the electrophoresed active agent are congruent with the blackened band of the 3H-DIFP labeled serine proteases representing the active principle of the agent.

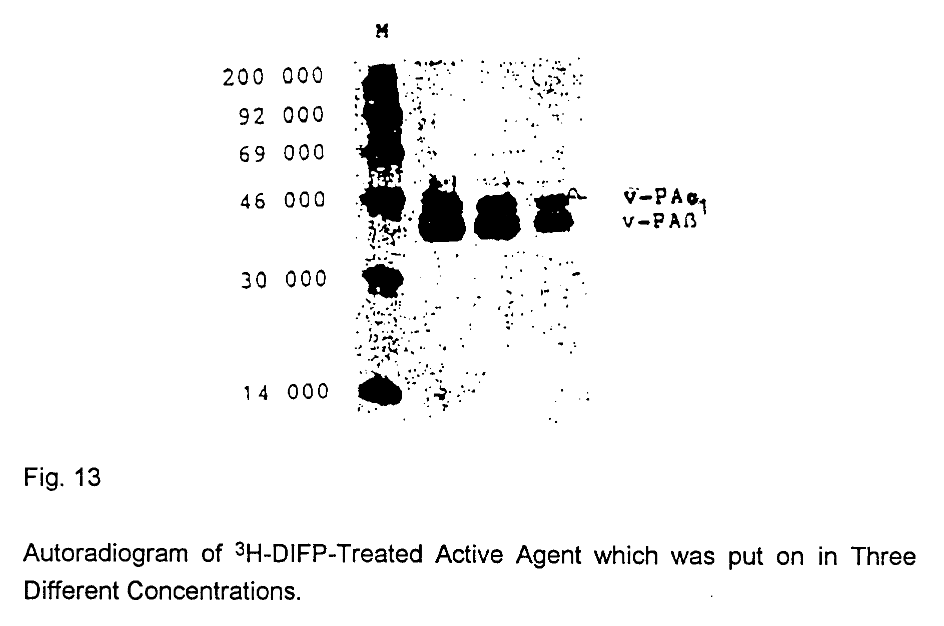

### Example 8

### Fibrin Binding of Various Plasminogen Activators and v-PA α₁ and v-PA β

In a specimen plan, 100 ml of plasminogenfree fibrinogen (2 mg/ml) in PBS / 0.01 % "Tween" 80, 10 »l of the corresponding plasminogen activators in various concentrations, and 10 »l of thrombin (0.3 U) are incubated at 37 °C for 10 minutes. Then the thus-formed fibrin is removed by centrifuging for 5 minutes at 10,000 g, and the residual plasminogen activator activity is determined in the supernatant by means of the fibrin plate testing method and brought into relation with the starting volume. In parallel therewith, under the same conditions, plasminogen activator solutions are prepared without fibrin (control; no binding). 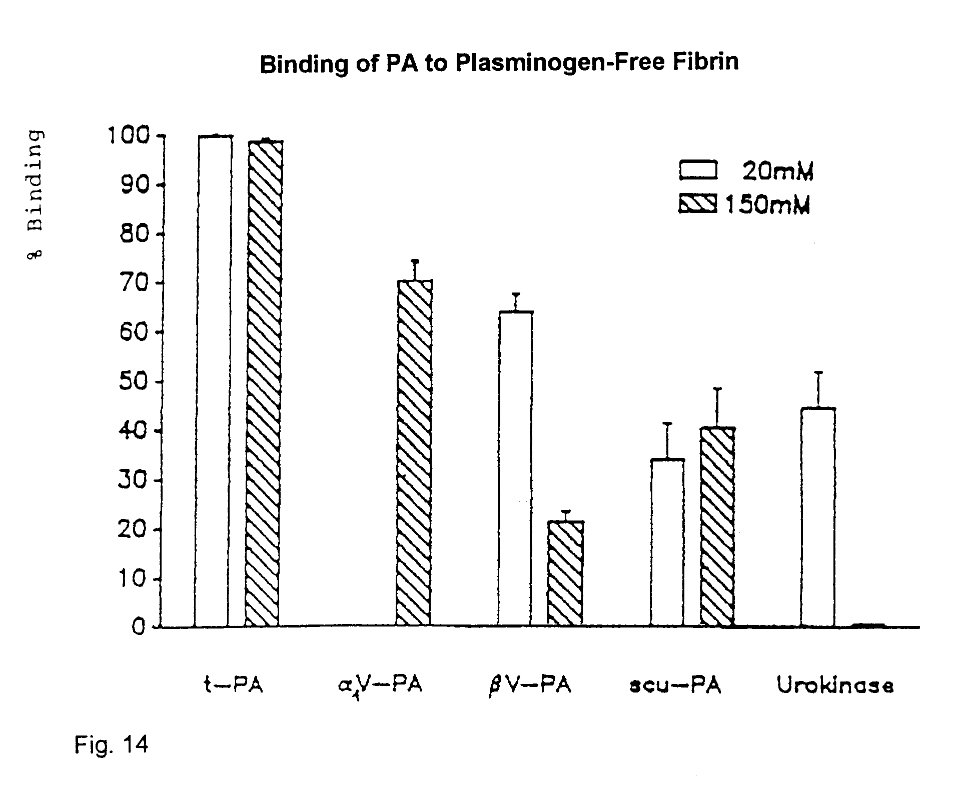
v-PA α₁ shows a similarly good fibrin binding as t-PA (Figure 14). In contrast thereto, v-PA β exhibits a substantially weaker fibrin affinity than v-PA α₁. This fibrin affinity displayed by v-PA β is furthermore strongly dependent on the NaCl concentration (Figure 14).

### Example 9

### Determination of the Km Value of Various Amidolytic Substrates with the Plasminogen Activators t-PA, Urokinase, and the Active Forms of v-PA α₁

In a 96-hole plate, respectively 90 »l of 100 mM Tris pH 8.0 / 100 mM NaCl / 0.01 % "Tween" 80 and 50 »l of the corresponding chromogenic substrate (all from Kabi Vitrum, Stockholm, Sweden) in the final concentrations of 0.03 / 0.06 / 0.1 / 0.3 / 0.6 and 1 mM are incubated with 10 »l (3 units per hole) of the corresponding plasminogen activator at 37 °C, and the thus-released p-nitroaniline is determined photometrically at 405 nm at various points in time between 0 - 7 hours. The resultant data are evaluated with the aid of a Lineweaver-Burk plot, and the Km value is determined (Segel, I.H., Enzyme Kinetics, John Wiley and Sons, New York). The following chromogenic substrates were utilized:
H-D-Val-Leu-Lys-pNA (S-2251)
H-D-Ile-Pro-Arg-pNA (S-2288)
< Glu-Gly-Arg-pNA (S-2444)
The following Km Values (mmol/l) were determined:

| **Plasminogen Activator** | **S-2288** | **S-2444** |
|---|---|---|
| t-PA | 0.7 (1.0) | 2.0 |
| Urokinase | 0.4 (0.2) | (0.1)(0.09) |
| v-PA α₁ | 1.0 | 2.0 |

The values indicated in parentheses are taken from the literature (Friedberger, P., in Scand. I. Clin. Lab. Invest. 42 - Supplement 1.62, 1982).

The active agent form of v-PA α₁ shows similar Km values as in case of t-PA. v-PA α₁ and t-PA show no conversion whatever with the chromogenic substrate S-2251.

### Example 10

### Isolation and Characterization of cDNA Clones with Segments for Plasminogen Activators from the Salivary Gland of the Vampire Bat Desmodus rotundus

1. Preparation of a cDNA Gene Bank
   Whole RNA from salivary glands of Desmodus rotundus is isolated according to the guanidinium isothiocyanate method [Chirgwin, J.M., Przybyla, A.E., MacDonald, R.J., Rutter, W.J., Biochemistry 18: 5294-5299 (1979)]. From 500 »g of whole RNA, 25 »g of poly A^{t}-RNA is isolated by oligo (dt) cellulose affinity chromatography [Aviv, H., Leder, P., Proc. Natl. Acad. Sci. USA 69 : 1408-1412 (1972)].
   cDNA synthesis takes place according to the method by Gubler and Hoffmann [Gubler, U., Hoffmann, B., Gene 25 : 263-269 (1983)] using the cDNA Synthex Kit of Pharmacia LKB Biotechnology AB. The individual steps take place exactly in accordance with the protocol indicated by the manufacturer. The double-strand cDNA provided with EcoRI adapters (/microgram) is linked with the EcoRI-cut, dephosphorylated phage vector Lambda gt 10 (4 »g) with the use of T4-DNA ligase [Huynh, T.V., Young, A., Davis, R.W. in Practical Approaches in Biochemistry. DNA Cloning Volume 1, ed. Glowen, D.M., IRL Oxford, Washington, DC (1985)]. The ligase batch is packaged into infectious phages, using packaging extracts (Gigapack II Gold Packaging extracts of Stratagene, La Jolla, USA) in accordance with the protocol indicated by the manufacturer. The thus-obtained primary cDNA gene bank contains 1 x 10⁶ recombined lambda phages.
2. Screening of the cDNA Gene Bank with a ³²P-Labeled Sequence of the cDNA Gene for the Human Tissue-Specific Plasminogen Activator (t-PA)
   A t-PA cDNA sequence [Fisher, R., Waller, E.K., Grossi, G., Thompson, D., Tizard, R., and Schleuning, W.-D., J. Biol. Chem. 260 : 11223 - 11230 (1985)] labeled radioactively by nick translation [Maniatis, T., Fritsch, E.F., Sambrook, J., Molecular Cloning, A Laboratory Manual (Cold Spring Harbor Laboratory, New York, 1982)] serves as a probe for the identification of vampire bat saliva plaminogen activator cDNA. The DNA of, in total, 50,000 recombined lambda phages is transferred to "Replika" membranes (Gene Screen Plus, trademark of Du Pont de Nemours, NEN Research Products).
   The treatment of the membranes and the composition of the hybridization solutions corresponds to the data provided by the manufacturer. The hybridization and washing temperature is 42 °C. The final washing step takes place in a 4 x SSC buffer. From the 50,000 screened recombined phages, 11 positive clones have been isolated.
3. Characterization of the cDNA of the Positive Clones
   The cDNA insert of the positive clones is trans-cloned according to standard methods into the commercially available sequencing vectors M13 mp 18 and M13 mp 19 [Messing, J., Meth. Enzymol. 101 : 20-769 (1983)] and sequenced according to the dideoxynucleotide method [Sanger, F., Nicklen, S., Coulson, A.R., Proc. Natl. Acad. Sci. USA 74 : 5463-5467] (1977). Comparison of the nucleotide sequences of the individual clones with the nucleotide sequence of the cDNA gene for the human t-PA [Pennica, D., Holmes, W.E., Kohr, W.J., Harkins, R.N., Vehar, G.A., Ward, C.A., Bennet, W.F., Yelverton, E., Seeburg, P.H., Heyneker, H.L. and Goeddel, D.V., Nature 303 : 214-221 (1983] yields a high degree of sequence identity (> 80 %). Also the derived protein sequences show a high sequence identity with parts of the human t-PA (> 70 %). None of the clones contains a cDNA for a complete vampire bat plasminogen activator gene. Partial sequences of one of these clones serve as a probe for screening a further cDNA gene bank from the salivary gland of Desmodus rotundus (Example 11).

### Example 11

### Isolation, Characterization and Sequencing of Complete cDNA Clones of the Plasminogen Activators from the Salivary Gland of Desmodus rotundus

1. Preparation of a cDNA Gene Bank from the Salivary Gland RNA of Desmodus rotundus
   A whole RNA is isolated from the salivary glands of vamipire bats (D. rotundus) by digestion with guanidinium isothiocyanate and subsequent ultracentrifuging by means of a cesium chloride cushion (1). From three milligrams of the lyophilized whole RNA, 80 »g of polyA⁺ RNA is obtained by twice performing affinity chromatography over oligo-deoxythymidine cellulose (2, 3). Five micrograms of this preparation is utilized for cDNA synthesis according to the method by Gubler and Hoffman (4) with several modifications ("ZAP-cDNA" Synthesis Kit, Stratagene) (5, 6). The synthesis of the first strand is initiated with the aid of an oligonucleotide containing an oligo-deoxythymidine proportion and the recognition sequence of the restriction endonuclease XhoI (6), and is carried out by reverse transcriptase of Mooney murine leukemia virus with the use of 5-methyldeoxycytidine. The second strand is synthetized by E. coli DNA polymerase I in the presence of E. coli ribonuclease H. The ends of the thus-formed, double strand cDNA are smoothed with T4 DNA polymerase and then linked to EcoRI adapters with the use of T4 DNA ligase. The resultant DNA ends are phosphorylated with the aid of T4 polynucleotide kinase. After digestion of the cDNA with the restriction enzyme XhoI, gel filtration is performed over "Sepharose" CL-4B (Pharmacia). The fractions containing cDNA with a minimum size of 500 base pairs are combined; the yield is about 1.2 »g of cDNA. One-third of this quantity is made to link with 3 »g of the EcoRI-XhoI restriction-digested and dephosphorylated arms of the bacteriophage vector Uni-ZAP (TM) XR (patent pending) of the company Stratagene (La Jolla, USA) (5, 7).
   The ligation batch is packaged into seven separate batches with the use of "Gigapack" II Gold Packaging extracts (Stratagene) (8). In this way, a primary cDNA gene bank is obtained with more than 4 x 10⁶ recombined lambda bacteriophages.
2. Identification of cDNA Clones of the Plasminogen Activators from Vampire Bat Saliva of Desmodus rotundus
   The probe employed for the identification of complete cDNA is a DNA fragment, produced by AluI-BamHI restriction digestion, of 76 base pairs in length (nucleotide 141-216 of vPA β) from the 5'-end of a clone isolated from the first cDNA gene bank (see Example 10). This fragment is labeled radioactively by nick translation (9) in the presence of [α-³²P] deoxyadenosine triphosphate and utilized for the hybridization of replica filters on which DNA of, in total, 1.2 x 10⁵ recombinant bacteriophages of the primary cDNA gene bank has been immobilized (10). Hybridization and washing temperature is 50 °C. The final washing step takes place in 2xSSC buffer (10). Of more than 200 clones that have emitted a signal in the autoradiography of the "Replika" filters, 50 clones are purified by separate plating out and repitition of the aforedescribed plaque filter hybridization. In this way, 38 clones are isolated which also in the second screening yield a clearly positive signal.
3. Characterization and Sequencing of the cDNA Clones
   The plasmid pBluescript SK, contained in the isolated positive UnitZAP (TM) XR bateriophage clones - inclusive of the cDNA fragment integrated in the same segment of the bacteriophage - is removed from the bacteriophages by so-called "in vivo excision" (7, 14), thus obtaining in total 35 different pBluescript SK plasmid clones stemming from the positive bacteriophage clones. The plasmids are isolated in accordance with the method by Birnboim and Doly (11), and their cDNA porportion is divided into four different classes α₁, α₂, β, γ, in accordance with restriction analysis with the enzymes BamHI and PstI (Figure 15) and DNA sequencing of the 5'-ends. The cDNA proportions of the longest cDNA clones of all four classes are subcloned into the bacteriophage vectors M13mp19 (12) and sequenced with the dideoxynucleotide method by Sanger ("Sequenase" kit United States Biochemical Corporation, Cleveland, USA) (13, 15) using, in part, oligonucleotide primers which are synthesized specifically for this purpose and are complementary to sequences of the cDNA proportions. The complete cDNA sequence (including a short segment of the polyA chains present at the 3'-end) with the thus-derived protein sequences are represented in Figure 16.

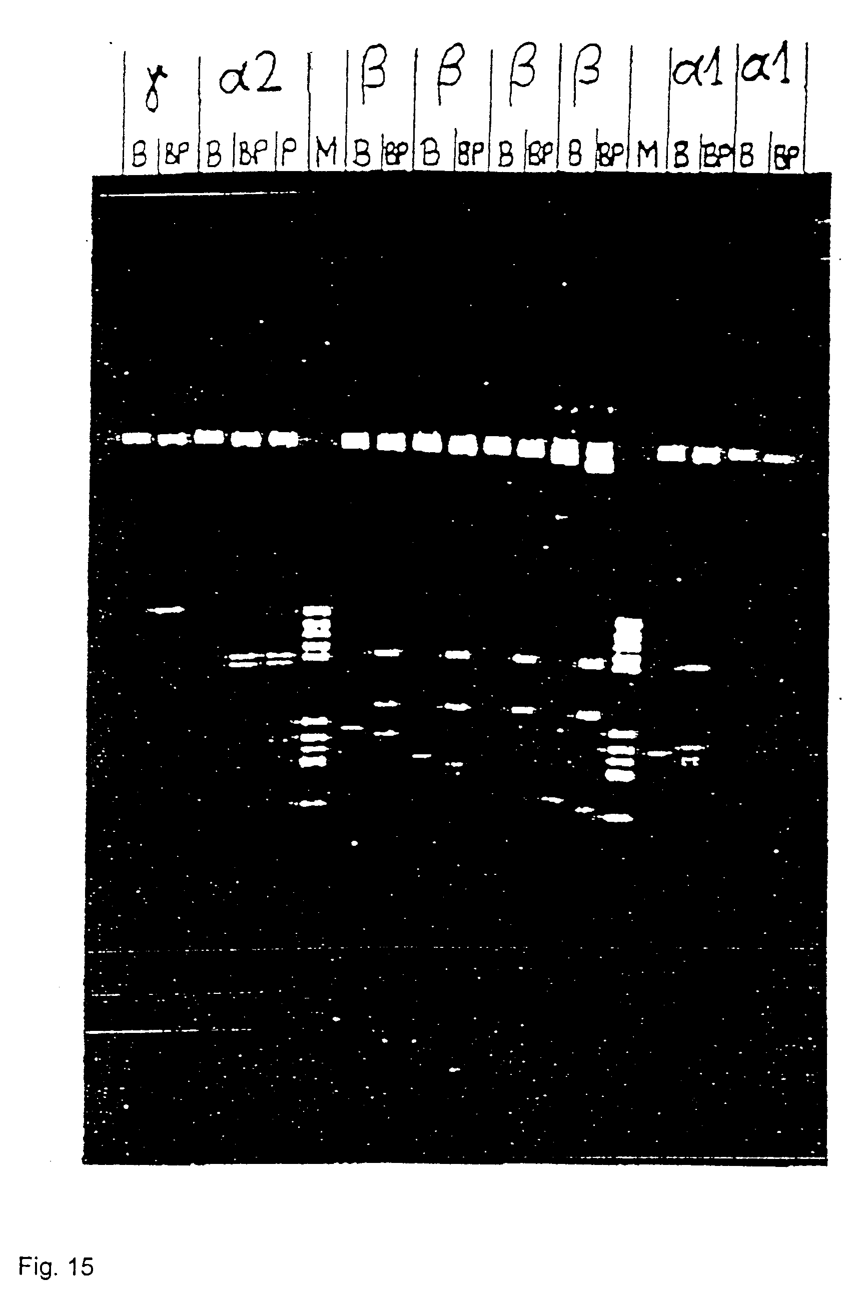

Restriction pattern of selected pBluescript SK plasmid clones with the respectively longest cDNA proportions from the four groups of alpha 1 (2 plasmid clones) alpha 2 (1 plasma clone), beta (4 plasmid clones) and gamma (1 plasmid clone). The plasmid DNA preparations, digested with the restriction enzymes BamHI ("B"), PstI ("P") or a mixture of these two restriction enzymes ("BP") were separated on a 1.8 % agarose gel. The yardstick regarding the size was represented by plasmid pBR322 ("M", DNA molecular weight marker 5 of the company Boehringer-Mannheim) digested with the restriction enzyme HaeIII. The size of the clearly visible fragments is 587,540, 504, 458, 434, 267, 234, 213, 192 to 184, and 123 base pairs (from the top toward the bottom). The agarose gel was coloured with ethidiumbromide after electrophoretic separation and photographed. From the groups alpha 1 and beta the left most pictured clones were sequenced, respectively.

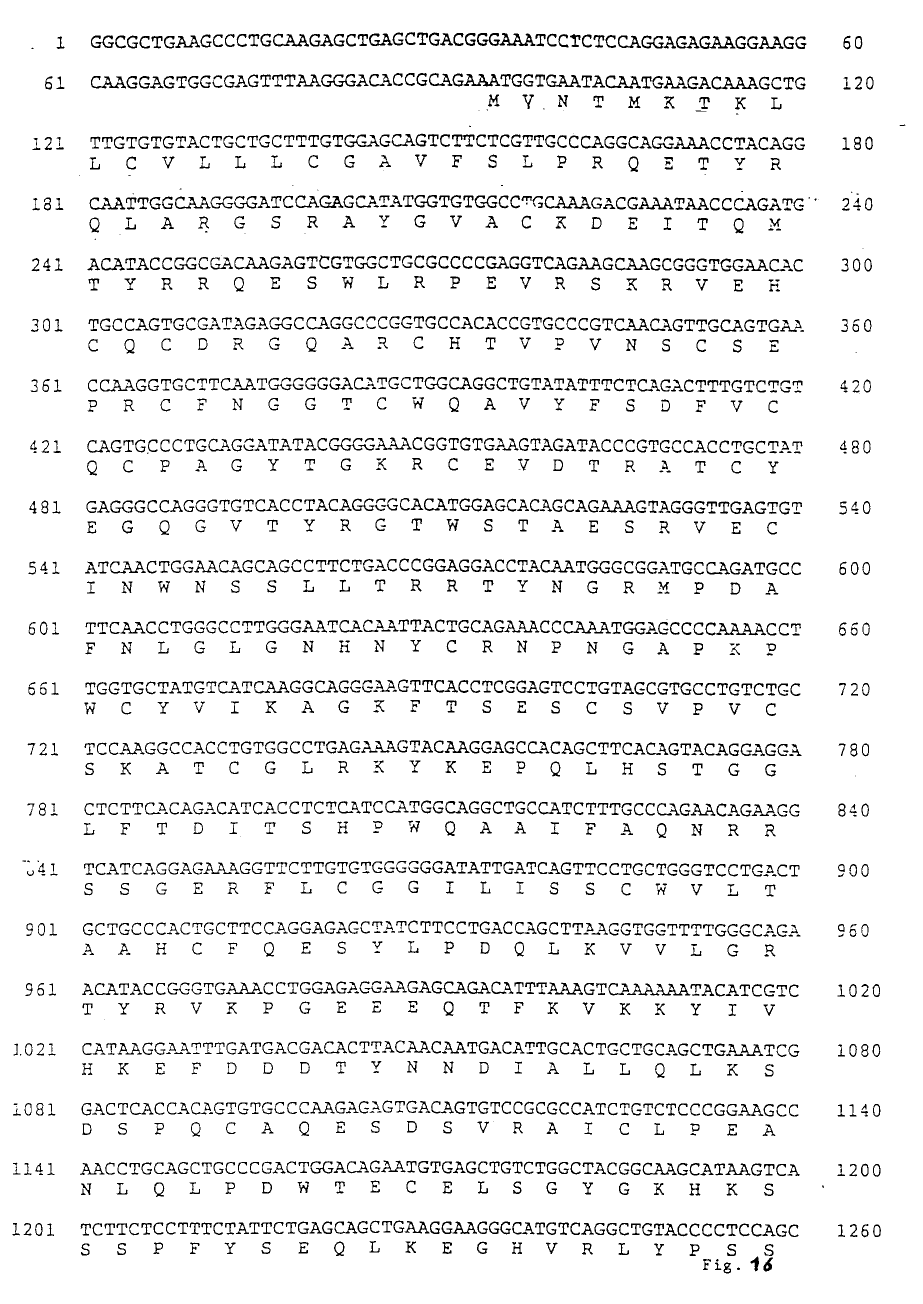

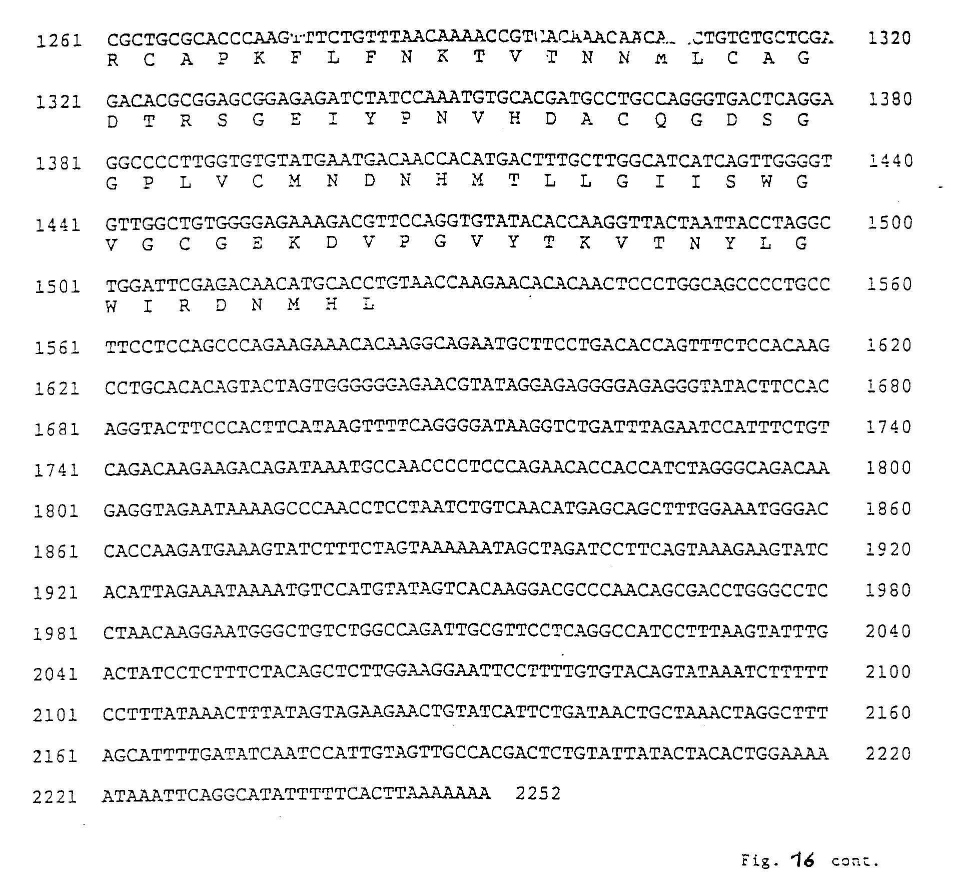

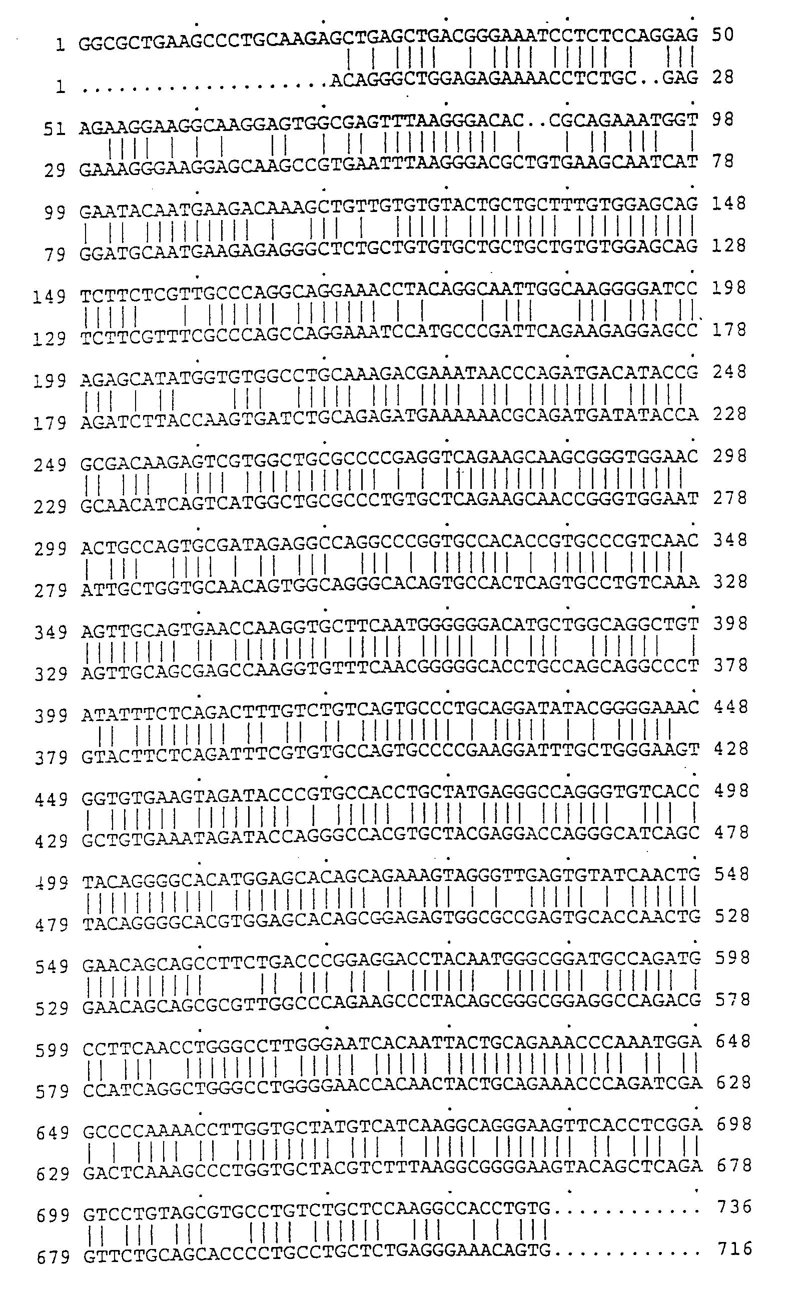

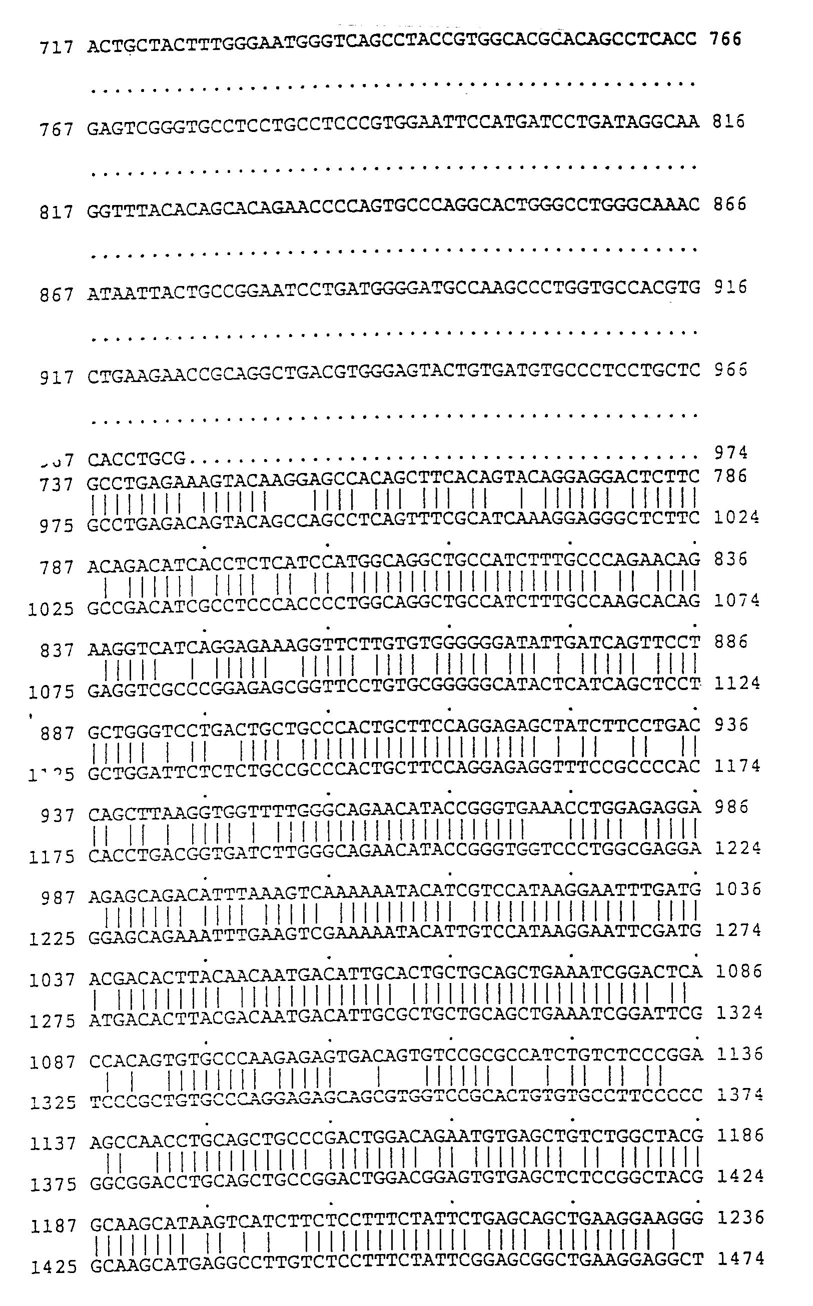

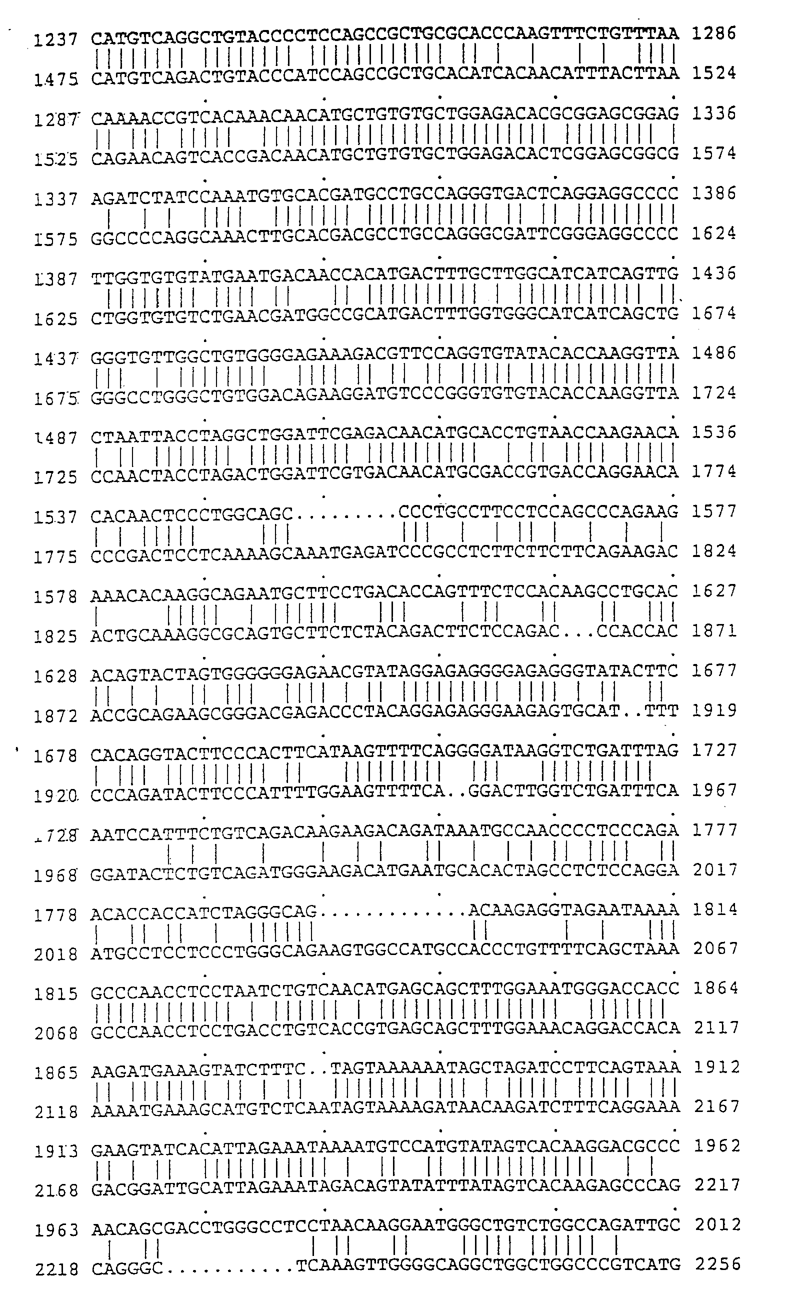

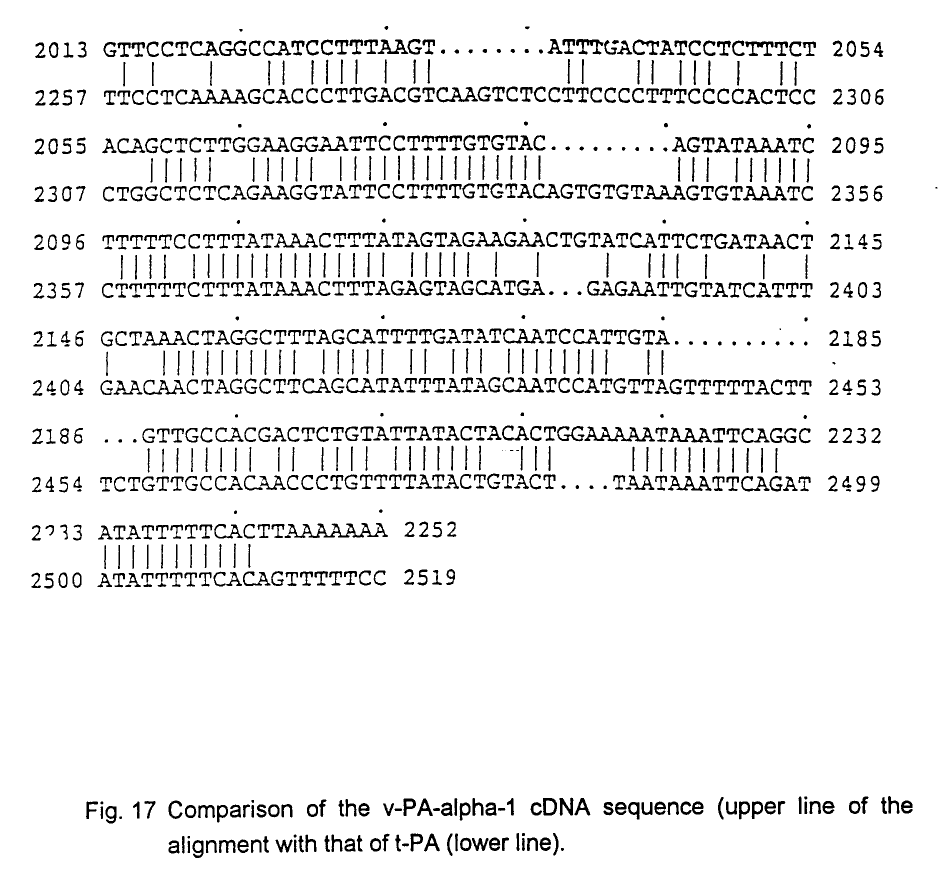

### Fig. 16

Complete DNA sequence of the cDNA insert of the longest found plasmid clone of the group alpha-1 with derived amino acid sequence. The coding sequence begins with nucleotide 94 (ATG ...) and ends with the stop codon at nucleotide 1527 (... TAA). The derived amino acid sequence is indicated in single-letter code, each amino acid standing under the first nucleotide of the triplet by which it is coded.

### Example 12

### Construction of Expression Vectors Containing the Coding Sequences of the Active Agent v-PA, and Testing of Same by Microinjection into Oocytes

The coding sequences from the cDNA clones of the v-PA form α₁ is obtained by cleavage with the restriction endonuclease EcoRI and, after treatment with the Klenow fragment of E. coli DNA polymerase I, and isolation from low melting temperature agarose gels, linked into the XbaI site of the vector pSVL-EcoRI filled up by the Klenow fragment of E. coli DNA polymerase I and treated with calf intestine phosphatase (Boehringer Mannheim).

The correct orientation is tested with BamHI. The pSVL-EcoRI involves a derivative of the commercially available SV40 expression plasmid pSVL (Pharmacia).

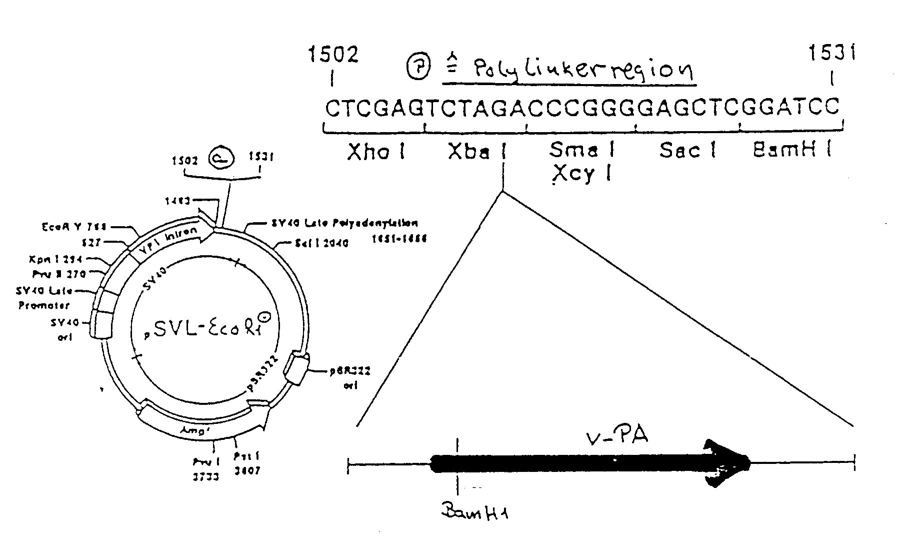

The thus-produced expression vectors are purified over a cesium chloride gradient and utilized for microinjection into Xenopus Iaevis oocytes.

Oocyte injection was performed according to the following cited articles:
1. Gurdon, J.B., and Wakefield, L., in "Microinjection and Organelle Transplantation Techniquees", eds. Celis, J.E., Graessmann, A., and Loyter, A., Academic Press, London, 1986.
2. Colman, A., in "Transcription and Translation - A Practical Approach", eds. Hames, R.J., and Higgins, S.J., IRL-Press Oxford, 1984.
3. Langer, G, "Analyse des Drosophila U1 SURNA Promotors in homologen in vitro und heterologen in vitro Transkriptionssystemen", Ph.D.Thesis, University of Kaiserslautern, 1987.

After embedding the oocytes, injected with the v-PA expression vectors, in a fibrin plate prepared with Barth medium (literature 2), lysis halos are produced. When using, in place of the fibrin plate, a casein plate, then no lysis takes place (α₁). Oocytes injected with a vector not containing the v-PA sequence do not exhibit a lysis halo.

The v-PA cDNA sequences contain a functional signal peptide sequence responsible for the export of the v-PA protein out of the cell.

These results clearly show that the thus-obtained v-PA cDNA clones are complete and, after incorporation into a suitable expression vector, produce active effective agent which is absolute fibrin-specific.

## Claims (Claims for the following Contracting State(s): AT, BE, CH, DE, DK, FR, GB, IT, LI, LU, NL, SE)

1. Substantially pure protein which is thrombolytic, activates plasminogen in the presence of fibrin but substantially not in the presence of fibrinogen, and is allosteric, said protein is a v-PAα1 having the amino acid sequence of Figure 16.

2. Isolated DNA encoding a protein according to the claim 1 having the nucleotide sequence of Figure 16.

3. A micro-organism transformed with a vector comprising DNA according to the claim 2.

4. A method of producing v-PAα1 comprising expressing DNA encoding v-PA α1 in the micro-organism according to the claim 3.

5. A substantially pure protein according to the claim 1 which has the N-terminal amino acid sequence Ala · Tyr · Gly · Val · Ala · ...

6. A substantially pure protein according to the claim 1 which has the N-terminal amino acid sequence Ala · Tyr · Gly · Val · Ala · Cys · Lys · Asp · Glu · Ile · Thr · Gln · Met · Thr · Tyr · Arg · ...

7. A medical agents based on the compounds according to any one of the claims 1, 2, 5 and 6 with customary auxiliary agents and excipients.

## Claims (Claims for the following Contracting State(s): ES)

1. A method of producing v-PAα1
comprising expressing DNA encoding v-PAα1 in the micro-organism transformed with a vector comprising DNA
encoding a protein having the amino acid sequence of Figure 16 wherein the protein is thrombolytic, activates plasminogen in the presence of fibrin but substantially not in the presence of fibrinogen, and is allosteric.

2. A method of producing v-PAα1
comprising expressing DNA encoding v-PAα1 in the micro-organism transformed with a vector comprising DNA
encoding a protein having the amino acid sequence of Figure 16 wherein the protein is thrombolytic, activates plasminogen in the presence of fibrin but substantially not in the presence of fibrinogen, and is allosteric and wherein the protein has the N-terminal amino acid sequence Ala · Tyr · Gly · Val · Ala · ...

3. Use of a compound produced and isolated by methods according to the claim 1 or 2 for the manufacture of a thrombolytic with customary auxiliary agents and excipients.

## Claims (Claims for the following Contracting State(s): GR)

1. Substantially pure protein which is thrombolytic, activates plasminogen in the presence of fibrin but substantially not in the presence of fibrinogen, and is allosteric, said protein is a v-PAα1 having the amino acid sequence of Figure 16.

2. Isolated DNA encoding a protein according to the claim 1 having the nucleotide sequence of Figure 16.

3. A micro-organism transformed with a vector comprising DNA according to the claim 2.

4. A method of producing v-PAα1 comprising expressing DNA encoding v-PA α1 in the micro-organism according to the claim 3.

5. A substantially pure protein according to the claim 1 which has the N-terminal amino acid sequence Ala · Tyr · Gly · Val · Ala · ...

6. A substantially pure protein according to the claim 1 which has the N-terminal amino acid sequence Ala · Tyr · Gly · Val · Ala · Cys · Lys · Asp · Glu · Ile · Thr · Gln · Met · Thr · Tyr · Arg · ...

7. Use of a compound according to any one of the claims 1, 2, 5 and 6 with customary auxiliary agents and excipients for manufacuture of a thrombolytic.

## Patentansprüche (Patentansprüche für folgende(n) Vertragsstaat(en): AT, BE, CH, DE, DK, FR, GB, IT, LI, LU, NL, SE)

1. Im wesentlichen reines Protein, das thrombolytisch ist, Plasminogen in Anwesenheit von Fibrin aktiviert, aber im wesentlichen nicht in Anwesenheit von Fibrinogen und welches allosterisch ist, besagtes Protein ist ein v-PAα1 mit der Aminosäuresequenz der Figur 16.

2. Isolierte DNA, kodierend ein Protein gemäß Anspruch 1, mit der Nukleotidsequenz der Figur 16.

3. Ein Mikroorganismus, transformiert mit einem Vektor, umfassend DNA gemäß Anspruch 2.

4. Eine Methode zur Herstellung von v-PAα1, umfassend die Expression von DNA, kodierend v-PAα1 im Mikroorganismus gemäß Anspruch 3.

5. Ein im wesentlichen reines Protein gemäß Anspruch 1, welches die N-terminale Aminosäuresequenz Ala · Tyr · Gly · Val · Ala · ... hat.

6. Ein im wesentlichen reines Protein gemäß Anspruch 1, welches die N-terminale Aminosäuresequenz Ala · Tyr · Gly · Val · Ala · Cys · Lys · Asp · Glu · Ile · Thr · Gln · Met · Thr · Tyr · Arg · ... hat.

7. Ein medizinisches Mittel, basierend auf einer Verbindung gemäß einem der Ansprüche 1, 2, 5 und 6, mit handelsüblichen Zusatz- und Trägerstoffen.

## Patentansprüche (Patentansprüche für folgende(n) Vertragsstaat(en): ES)

1. Eine Methode zur Herstellung von v-PAα1, umfassend die Expression von DNA, kodierend v-PAα1 im Mikroorganismus, transformiert mit einem Vektor, umfassend DNA, die ein Protein mit der Aminsoäuresequenz der Figur 16 kodiert, worin das Protein thrombolytisch ist, Plasminogen in Anwesenheit von Fibrin aktiviert, aber im wesentlichen nicht in Anwesenheit von Fibrinogen und welches allosterisch ist.

2. Eine Methode zur Herstellung von v-PAα1, umfassend die Expression von DNA, kodierend v-PAα1 im Mikroorganismus, transformiert mit einem Vektor, umfassend DNA, die ein Protein mit der Aminosäuresequenz der Figur 16 kodiert, worin das Protein thromboly-tisch ist, Plasminogen in Anwesenheit von Fibrin aktiviert, aber im wesentlichen nicht in Anwesenheit von Fibrinogen und welches allosterisch ist, und worin das Protein die N-terminale Aminosäuresequenz Ala · Tyr · Gly · Val · Ala · ... hat.

3. Verwendung einer Verbindung, hergestellt und isoliert mit Methoden gemäß den Ansprüchen 1 oder 2, zur Herstellung eines Thrombolytikums mit handelsüblichen Zusatz- und Trägerstoffen.

## Patentansprüche (Patentansprüche für folgende(n) Vertragsstaat(en): GR)

1. Im wesentlichen reines Protein, das thrombolytisch ist, Plasminogen in Anwesenheit von Fibrin aktiviert, aber im wesentlichen nicht in Anwesenheit von Fibrinogen und welches allosterisch ist, besagtes Protein ist ein v-PAα1 mit der Aminosäuresequenz der Figur 16.

2. Isolierte DNA, kodierend ein Protein gemäß Anspruch 1, mit der Nukleotidsequenz der Figur 16.

3. Ein Mikroorganismus, transformiert mit einem Vektor, umfassend DNA gemäß Anspruch 2.

4. Eine Methode zur Herstellung von v-PAα1, umfassend die Expression von DNA, kodierend v-PAα1 im Mikroorganismus gemäß Anspruch 3.

5. Ein im wesentlichen reines Protein gemäß Anspruch 1, welches die N-terminale Aminosäuresequenz Ala · Tyr · Gly · Val · Ala · ... hat.

6. Ein im wesentlichen reines Protein gemäß Anspruch 1, welches die N-terminale Aminosäuresequenz Ala · Tyr · Gly · Val · Ala · Cys · Lys · Asp · Glu · Ile · Thr · Gln · Met · Thr · Tyr · Arg · ... hat.

7. Verwendung einer Verbindung gemäß einem der Ansprüche 1, 2, 5 und 6, mit handelsüblichen Zusatz- und Trägerstoffen, zur Herstellung eines Thrombolytikums.

## Revendications (Revendications pour l'(les) Etat(s) contractant(s) suivant(s): AT, BE, CH, DE, DK, FR, GB, IT, LI, LU, NL, SE)

1. Protéine substantiellement pure qui est thrombolytique, qui active le plasminogène en présence de la fibrine, mais substantiellement non en présence du fibrinogène, et qui est allostérique, ladite protéine est un v-PAα1 contenant la séquence d'acides aminés de la figure 16.

2. ADN isolé codant pour une protéine selon la revendication 1, contenant la séquence de nucléotides de la figure 16.

3. Micro-organisme transformé a l'aide d'un vecteur comprenant l'ADN selon la revendication 2.

4. Méthode de préparation de v-PAα1 comprenant l'expression de l'ADN codant pour le v-PAα1 dans le micro-organisme selon la revendication 3.

5. Protéine substantiellement pure selon la revendication 1, qui contient à l'extrémité 5' la séquence d'acides nucléiques Ala-Tyr-Gly-Val-Ala-...

6. Protéine substantiellement pure selon la revendication 1, qui contient à l'extrémité 5' la séquence d'acides aminés Ala-Tyr-Gly-Val-Ala-Cys-Lys-Asp-Glu-Ile-Thr-Gln-Met-Thr-Tyr-Arg-...

7. Agents médicaux a base des composés selon l'une quelconque des revendications 1, 2, 5 et 6 avec des adjuvants et excipients usuels.

## Revendications (Revendications pour l'(les) Etat(s) contractant(s) suivant(s): ES)

1. Méthode de préparation de v-PAα1
comprenant l'expression de l'ADN codant pour v-PAα1 dans le micro-organisme transformé à l'aide d'un vecteur comprenant l'ADN
codant pour une protéine contenant une séquence d'acides aminés de la figure 16 où la protéine est thrombolytique, active le plasminogène en présence de la fibrine, mais substantiellement non en présence du fibrinogène et est allostérique.

2. Méthode de préparation de v-PAα1
comprenant l'expression de l'ADN codant pour v-PAα1 dans le micro-organisme transformé à l'aide d'un vecteur comprenant l'ADN
codant pour une protéine contenant la séquence d'acides aminés de la figure 16, où la protéine est thrombolytique, active le plasminogène en présence de la fibrine, mais substantiellement non en présence du fibrinogène et est allostérique et où la protéine contient à l'extrémité 5' la séquence d'acides aminés Ala-Tyr-Gly-Val-Ala-...

3. Utilisation d'un composé préparé et isolé à l'aide des méthodes selon la revendication 1 ou 2 pour la préparation d'un thrombolytique avec des adjuvants et excipients usuels.

## Revendications (Revendications pour l'(les) Etat(s) contractant(s) suivant(s): GR)

1. Protéine substantiellement pure qui est thrombolytique, qui active le plasminogène en présence de la fibrine, mais substantiellement non en présence du fibrinogène, et qui est allostérique, ladite protéine est un v-PAα1 contenant la séquence d'acides aminés de la figure 16.

2. ADN isolé codant pour une protéine selon la revendication 1, contenant la séquence de nucléotides de la figure 16.

3. Micro-organisme transformé à l'aide d'un vecteur comprenant l'ADN selon la revendication 2.

4. Méthode de préparation de v-PAα1 comprenant l'expression de l'ADN codant pour le v-PAα1 dans le micro-organisme selon la revendication 3.

5. Protéine substantiellement pure selon la revendication 1, qui contient à l'extrémité 5' la séquence d'acides nucléiques Ala-Tyr-Gly-Val-Ala-...

6. Protéine substantiellement pure selon la revendication 1, qui contient à l'extrémité 5' la séquence d'acides aminés Ala-Tyr-Gly-Val-Ala-Cys-Lys-Asp-Glu-Ile-Thr-Gln-Met-Thr-Tyr-Arg-...

7. Utilisation d'un composé selon l'une quelconque des revendications 1, 2, 5 et 6 avec des adjuvants et excipients usuels dans la fabrication d'un thrombolytique.
